# EUROPÄISCHE PATENTANMELDUNG

(11) **EP 2 689 745 A1**
(43) Veröffentlichungstag der Anmeldung: **29.01.2014**
(21) Anmeldenummer: 13173362.8
(22) Anmeldetag: 24.06.2013
(51) Int. Cl.: A61C 5/00, A61B 17/70

(54) **Skelettale Verankerungsvorrichtung**

(30) Priorität: 23.07.2012 DE 102012014527
(71) Anmelder: Pasin, Enrico, 83435 Bas Reichenhall (DE)
(72) Erfinder: Pasin, Enrico, 83435 Bas Reichenhall (DE)
(74) Vertreter: Kilian Kilian & Partner

(57) **Zusammenfassung**

Die erfindungsgemäße skelettale Verankerungsvorrichtung zur Befestigung zumindest eines kieferorthopädischen Elements zur Verwendung für eine kieferorthopädische Behandlung umfasst ein Verankerungselement (10), das einen Verankerungsabschnitt (12) aufweist, der eingerichtet ist, skelettal befestigt zu werden, und weiterhin einen Befestigungsabschnitt (14) aufweist, der eingerichtet ist, mit dem zumindest einen kieferorthopädischen Element gekoppelt zu werden.

Erfindungsgemäß ist vorgesehen, dass der Befestigungsabschnitt (14) mit einer Befestigungseinrichtung vorgesehen ist, die derart ausgebildet ist, dass das kieferorthopädische Element zumindest abschnittsweise mit kontinuierlich einstellbarer Klemmkraft an dem Befestigungsabschnitt (14) verklemmt werden kann.

## Beschreibung

Die Erfindung betrifft eine skelettale Verankerungsvorrichtung zur Befestigung zumindest eines kieferorthopädischen Elements zur Verwendung für eine kieferorthopädische Behandlung, wobei die Verankerungsvorrichtung ein Verankerungselement umfasst, das einen Verankerungsabschnitt aufweist, der eingerichtet ist, skelettal, d.h. an einem Knochen des Menschen, beispielsweise einen Ober- oder Unterkieferknochen, befestigt zu werden, und weiterhin einen Befestigungsabschnitt aufweist, der eingerichtet ist, mit dem zumindest einen kieferorthopädischen Element gekoppelt zu werden.

Bekannte skelettale Verankerungsvorrichtungen umfassen beispielsweise in Form von kieferorthopädischen Knochenschrauben ausgebildete Verankerungselemente, wobei solche kieferorthopädischen Knochenschrauben oftmals nur temporär skelettal verankert werden.

Unter den verschiedenen skelettalen Verankerungssystemen bzw. -vorrichtungen haben sich aufgrund ihrer geringen chirurgischen Invasivität und der relativ geringen Kosten weitgehend kieferorthopädische Knochenschrauben durchgesetzt, welche in Fachkreisen auch als sogenannte Mini-Pins, Mini-Implantate, etc. bezeichnet werden.

Beispielsweise ist ein Orthosystem von Straumann® als kieferorthopädisches Implantatsystem bekannt, welches ein zweiteiliges System ist, dessen Implantat zumindest abschnittsweise eine aktive angeraute Implantatoberfläche aufweist, welche im implantierten Zustand osseointegriert (verknöchert). Dieses Implantatsystem ist insoweit problembehaftet, als sich das in Form einer Knochenschraube ausgebildete Implantat nicht mehr aufgrund der Osseointegration herausdrehen lässt. Wie bei einem klassischen dentalen Implantatsystem können sog. Abutments bzw. Aufsätze unterschiedlicher Art zur Kopplung des kieferorthopädischen Elements für die kieferorthopädische Behandlung bei diesem System auf das eingeschraubte bzw. eingesetzte Implantat, d.h. auf die Knochenschraube, aufgeschraubt werden. Demnach fungiert das Abutment als ein Zwischenglied zwischen dem Knochenschraubenkopf und dem kieferorthopädischen Element für die kieferorthopädische Behandlung.

Neben dem hohen finanziellen, chirurgischen und organisatorischen Aufwand dieses Orthosystems muss zudem eine Einheilzeit von ca. drei Monaten abgewartet werden.

Deshalb haben sich zahlreiche einteilige, temporäre, kieferorthopädische, selbstschneidende, selbstbohrende Knochenschrauben, sogenannte Mini-Pins, Mini-Implantate, mit polierter Implantatoberfläche, beispielsweise die Dual-Top®-Schraube von Promedia, in der Kieferorthopädie durchgesetzt. Vorteilhaft an einer derartigen kieferorthopädischen Knochenschraube ist der geringe chirurgische Aufwand bei deren Einsatz. Auch die Entfernung dieser einteiligen Knochenschraube ist deutlich einfacher, da diese nicht osseointegriert (verknöchert) und sich somit leicht herausdrehen bzw. entfernen lässt.

Da diese Art von Knochenschraube grundsätzlich einteilig ist, unterscheidet man diese Knochenschrauben nach den verschiedenen Gestaltungsformen des Schraubenkopfes. An diesen verschiedenen Schraubenköpfen können dann die kieferorthopädischen Elemente für die kieferorthopädische Behandlung befestigt werden. Die Befestigung des kieferorthopädischen Elements für die kieferorthopädische Behandlung an dem Schraubenkopf erfolgt üblicherweise über eine Steckverbindung, die mittels eines Spanndrahts, einer Drahtligatur oder einer Gummiligatur gesichert wird. Bei Steckverbindungen des kieferorthopädischen Elements für die kieferorthopädische Behandlung mit dem Schraubenkopf der Knochenschraube ist die Sicherheit der Verbindung problematisch, da sich diese unter Umständen leicht lösen kann.

Bei dem BENEFIT-System von Mondeal® wird eine zweiteilige Knochenschraube verwendet. Diese osseointegriert nicht, da diese eine vollständig polierte Oberfläche besitzt. Charakteristisch für diese Kinochenschraube ist die Schraubverbindung von Knochenschraube bzw. Knochenschraubenkopf mit dem Abutment bzw. Aufsatz zur Kopplung des kieferorthopädischen Elements für die kieferorthopädische Behandlung. Das Abutment bzw. der Aufsatz wird bei diesem System mittels einer Fixierschraube an dem Knochenschraubenkopf befestigt. Insbesondere wird das Abutment bzw. der Aufsatz bei diesem System durch eine Platte oder eine Kappe gebildet, die mittels der zusätzlichen Fixierschraube an dem Knochenschraubenkopf befestigt wird. Beispielsweise kann die Verbindung des kieferorthopädischen Elements für die kieferorthopädische Behandlung mit dem Abutment durch eine Schweißverbindung oder eine Steckverbindung hergestellt werden. Im Falle einer Steckverbindung wird das kieferorthopädische Element für die kieferorthopädische Behandlung mittels eines Spanndrahts, einer Drahtligatur oder Gummiligatur gesichert. Herstellungsbedingt ist dieses System jedoch äußerst aufwändig und somit kostspielig.

Der Erfindung liegt daher die Aufgabe zugrunde, die Nachteile der dem Stand der Technik angehörenden Verankerungsvorrichtungen, insbesondere der bekannten Knochenschrauben, zumindest teilweise zu überwinden. Insbesondere ist es Aufgabe der Erfindung, eine kostengünstige kieferorthopädische Verankerungsvorrichtung zur Verfügung zu stellen, deren Einsatz und Entfernung im Vergleich zum Stand der Technik mit geringerem Aufwand erfolgen kann.

Diese Aufgabe wird durch eine skelettale Verankerungsvorrichtung gemäß Anspruch 1 gelöst. Vorteilhafte Ausgestaltungen und Ausführungsformen der Erfindung sind Gegenstand der abhängigen Ansprüche.

Die erfindungsgemäße skelettale Verankerungsvorrichtung zur Befestigung zumindest eines kieferorthopädischen Elements zur Verwendung für eine kieferorthopädische Behandlung umfasst ein Verankerungselement, das einen Verankerungsabschnitt aufweist, der eingerichtet ist, skelettal an einem Ober- oder Unterkieferknochen des Menschen befestigt zu werden, und weiterhin einen Befestigungsabschnitt aufweist, der eingerichtet ist, mit dem zumindest einen kieferorthopädischen Element, vorzugsweise unmittelbar bzw. direkt, gekoppelt zu werden, wobei der Befestigungsabschnitt mit einer Befestigungseinrichtung vorgesehen ist, die derart ausgebildet ist, dass das kieferorthopädische Element zumindest abschnittsweise mit kontinuierlich einstellbarer Klemmkraft an dem Befestigungsabschnitt verklemmt werden kann. Vorzugsweise ist der Verankerungsabschnitt des als Knochenschraube ausgebildeten Verankerungselements mit einer teil-semiaktiven Oberfläche vorgesehen, so dass die Eigenschaft und Möglichkeit, die Schraube bei Bedarf einfach herauszudrehen, nicht verlorengeht. Ein derartiges als Knochenschraube ausgebildetes Verankerungselement ist insbesondere dazu vorgesehen, kieferorthopädische Elemente zur Verwendung für eine kieferorthopädische Behandlung, beispielsweise Drahtelemente oder Federn, direkt bzw. unmittelbar, d.h. ohne zwischengeschaltetes Abutment (bzw. Aufsatz), zu haltern, beispielsweise mittels einer Verklemmung. Daher ist das in Form einer Knochenschraube ausgebildete Verankerungselement einteilig ausgebildet, so dass das kieferorthopädische Element zur Verwendung für eine kieferorthopädische Behandlung nicht über das Abutment bzw. den Aufsatz aufgeschraubt werden muss, sondern direkt aufgesteckt wird und beispielsweise mittels eines als Schraubenmutter ausgebildeten Befestigungselements gesichert wird. Die Schraubenmutter erzeugt eine sichere Verklemmung des kieferorthopädischen Elements zur Verwendung für eine kieferorthopädische Behandlung bei einstellbarer Klemmkraft und ist damit nicht über einen separaten Spanndraht oder ein Gummiband zu befestigen. Vorzugsweise können kieferorthopädische Elemente zur Verwendung für eine kieferorthopädische Behandlung wie Rund- oder Vierkantdrähte an dem Befestigungsabschnitt der erfindungsgemä-βen Verankerungsvorrichtung verklemmt werden. Runddrähte haben dabei vorzugsweise einen Durchmesser von in etwa 0,6 mm bis 1,1 mm, während üblicherweise Vierkantdrähte Abmessungen von nicht mehr als etwa 0,9 mm x 0,9 mm aufweisen. Insbesondere ist vorgesehen, den Runddraht oder Vierkantdraht direkt an dem als Knochenschraube ausgebildeten Verankerungselement, insbesondere an dem Schraubenkopf, mittels einer verschraubbaren Verbindung, welche eine Verklemmung erzeugen kann, zu befestigen. Ein Zwischenglied wie beispielsweise das vorgenannte Abutment ist somit nicht mehr notwendig. Der implantierbare Teil der Knochenschraube, d.h. deren Verankerungsabschnitt, wird vorzugsweise durch den Schraubenschaft mit geeigneten Außengewinde ausgebildet, der ein bereits bewährtes selbstschneidendes, selbstbohrendes Gewinde mit einer teil-semiaktiven Oberfläche aufweisen kann.

Die erfindungsgemäße skelettale Verankerungsvorrichtung kann in vorteilhafterweise derart weitergebildet werden, dass die Befestigungseinrichtung derart ausgebildet ist, dass das kieferorthopädische Element zwischen einem Aufnahmeabschnitt des Befestigungsabschnitts und einem mit dem Befestigungsabschnitt koppelbaren Befestigungselement verklemmt werden kann, wobei der Aufnahmeabschnitt und das Befestigungselement so ausgebildet sind, dass in Abhängigkeit von einer Änderung von deren relativer Lage zueinander die Klemmkraft kontinuierlich eingestellt werden kann.

Weiterhin kann die erfindungsgemäße skelettale Verankerungsvorrichtung so verwirklicht werden, dass der Aufnahmeabschnitt und das mit dem Befestigungsabschnitt koppelbare Befestigungselement so ausgebildet sind, dass die Klemmkraft durch Drehung des Aufnahmeabschnitts relativ zu dem Befestigungselement kontinuierlich eingestellt werden kann.

Ferner kann die erfindungsgemäße skelettale Verankerungsvorrichtung derart umgesetzt werden, dass die Verankerungsvorrichtung ein in Form einer Knochenschraube ausgebildetes Verankerungselement umfasst, das einen in Form eines Schraubenschafts ausgebildeten Verankerungsabschnitt aufweist, der eingerichtet ist, skelettal, vorzugsweise mittels eines Außengewindes, befestigt zu werden, und weiterhin einen in Form eines an den Schraubenschaft angrenzenden Schraubenkopfs ausgebildeten Befestigungsabschnitt aufweist, der eingerichtet ist, mit dem zumindest einen kieferorthopädischen Element gekoppelt zu werden, wobei der in Form des Schraubenkopfes ausgebildete Befestigungsabschnitt mit einer Befestigungseinrichtung vorgesehen ist, die derart ausgebildet ist, dass das kieferorthopädische Element zumindest abschnittsweise zwischen einer als Aufnahmeabschnitt fungierenden Aussparung des Schraubenkopfs und einer auf ein Außengewinde des Schraubenkopfs aufgeschraubten und als Befestigungselement fungierenden Mutter mit kontinuierlich einstellbarer Klemmkraft verklemmt werden kann.

Darüber hinaus kann die erfindungsgemäße skelettale Verankerungsvorrichtung so realisiert werden, dass die Aussparung in dem Schraubenkopf vorzugsweise als durch den Schraubenkopf durchgängiger Schlitz ausgebildet ist und/oder die Aussparungstiefenrichtung parallel zur Schraubenachse verläuft und/oder die Aussparung geradlinig durch die Schraubenachse verläuft.

Eine bevorzugte Ausführungsform der Erfindung wird nachfolgend anhand der Figuren beispielhaft erläutert.

Es zeigen:
Fig. 1 eine perspektivische Darstellung einer erfindungsgemäßen kieferorthopädischen Verankerungsvorrichtung,
Fig. 2 eine Darstellung einer Schnittansicht der erfindungsgemä-βen kieferorthopädischen Verankerungsvorrichtung von Fig. 1 im Längsschnitt,
Fig. 3 eine weitere Darstellung der erfindungsgemäßen kieferorthopädischen Verankerungsvorrichtung von Fig. 1,
Fig. 4 eine weitere Darstellung der erfindungsgemäßen kieferorthopädischen Verankerungsvorrichtung von Fig. 1 in einer Draufsicht, und
Fig. 5 eine perspektivische Darstellung eines Befestigungselements der erfindungsgemäßen kieferorthopädischen Verankerungsvorrichtung von Fig. 1.

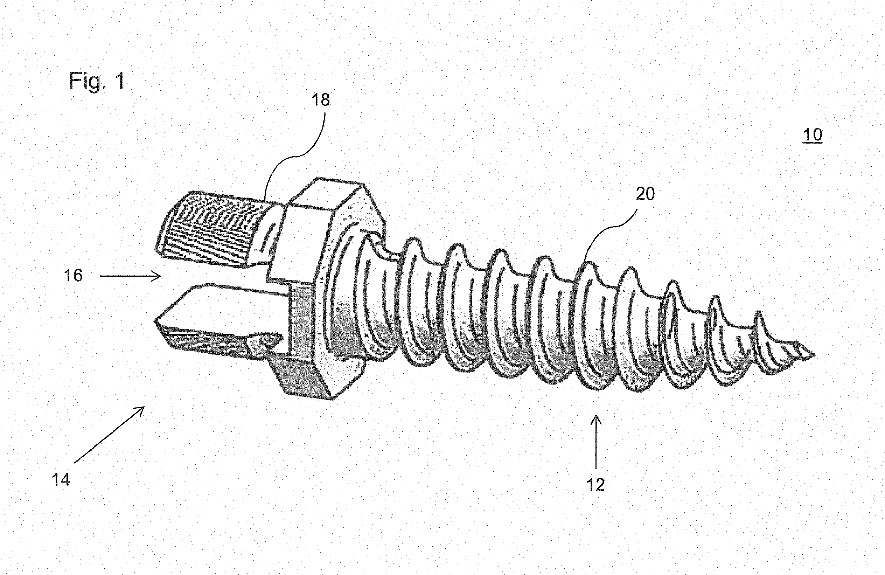

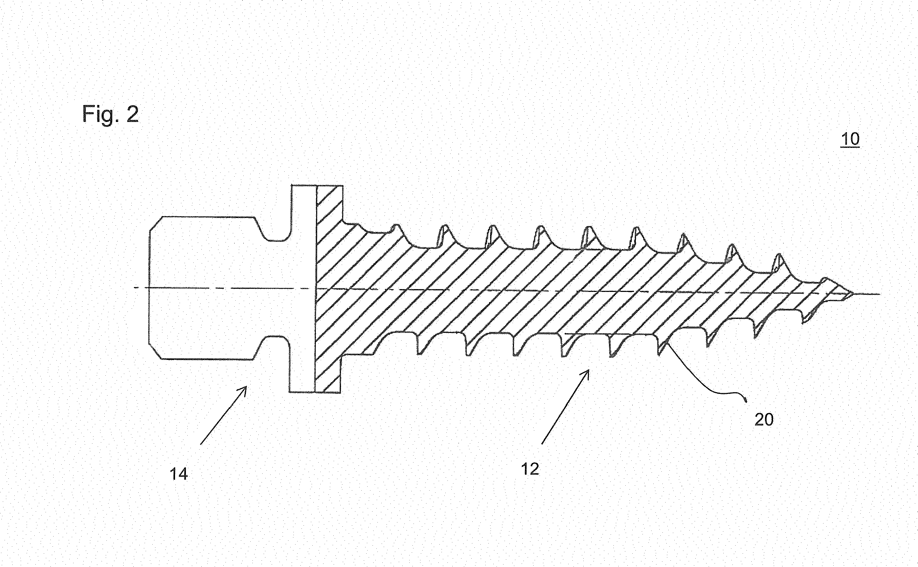

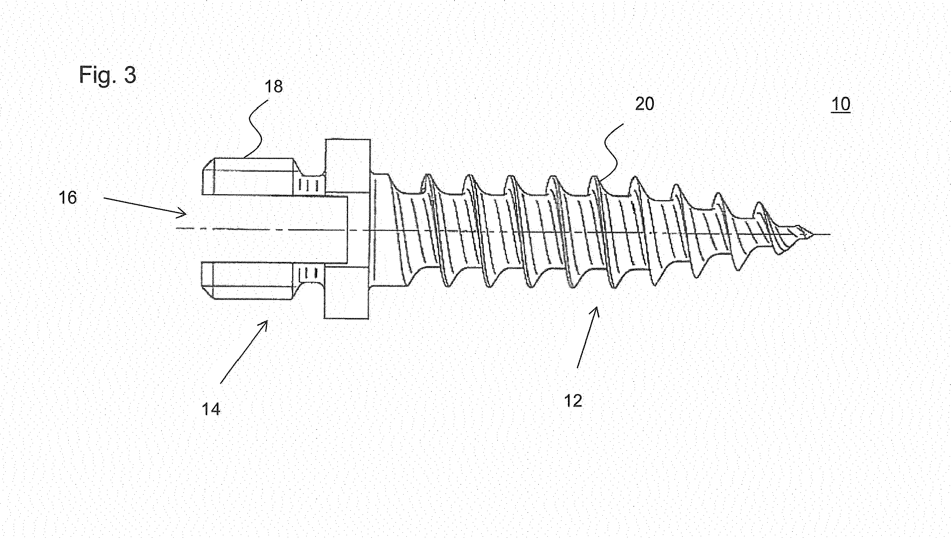

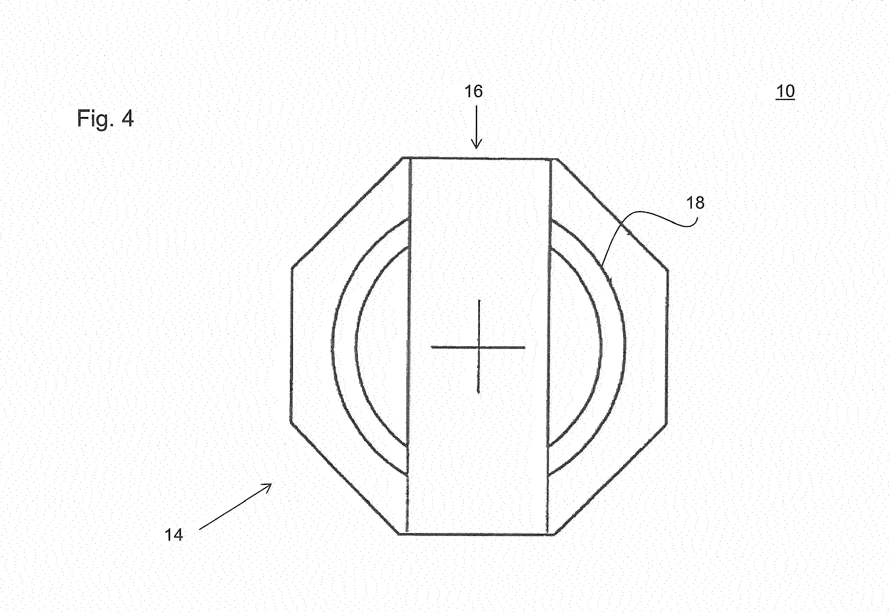

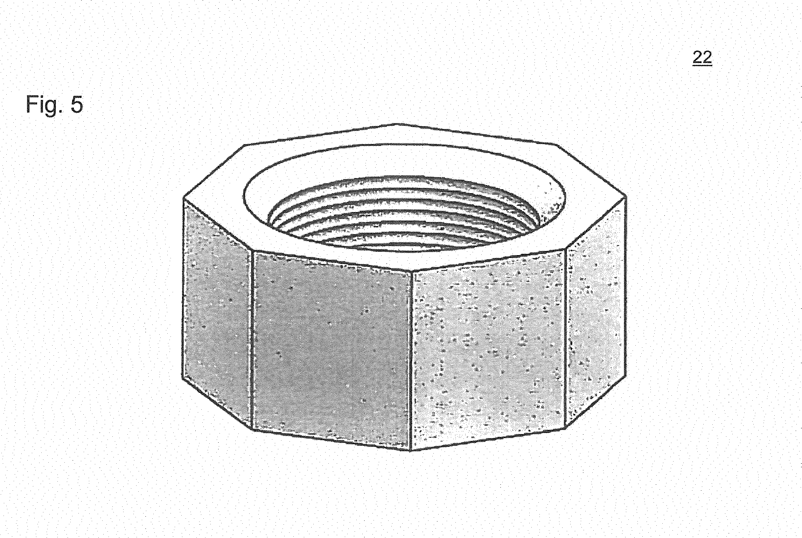

In den Figuren 1 bis 4 ist eine erfindungsgemäße skelettale Verankerungsvorrichtung zur Befestigung zumindest eines kieferorthopädischen Elements zur Verwendung für eine kieferorthopädische Behandlung gezeigt. Wie insbesondere aus den Figuren 1 bis 4 ersichtlich ist, umfasst die erfindungsgemäße Verankerungsvorrichtung ein Verankerungselement 10, das einen Verankerungsabschnitt 12 aufweist, der eingerichtet ist, skelettal, beispielsweise an einem Unter- oder Oberkieferknochen, befestigt zu werden. Weiterhin weist das Verankerungselement 10 einen Befestigungsabschnitt 14 auf, der eingerichtet ist, mit dem zumindest einen kieferorthopädischen Element zur kieferorthopädischen Behandlung gekoppelt zu werden. Beispielweise kann das kieferorthopädische Element durch einen Rund- oder Vierkantzahnspangendraht ausgebildet werden. Ferner ist der Befestigungsabschnitt 14 mit einer Befestigungseinrichtung vorgesehen, die derart ausgebildet ist, dass das kieferorthopädische Element zumindest abschnittsweise mit kontinuierlich einstellbarer Klemmkraft an dem Befestigungsabschnitt 14 verklemmt werden kann. Insbesondere ist die Befestigungseinrichtung derart ausgebildet, dass das kieferorthopädische Element zwischen einem Aufnahmeabschnitt 16 des Befestigungsabschnitts 14 und einem mit dem Befestigungsabschnitt 14 koppelbaren Befestigungselement 22 (siehe Fig. 5) verklemmt werden kann, wobei der Aufnahmeabschnitt 16 und das Befestigungselement 22 so ausgebildet sind, dass in Abhängigkeit von einer Änderung von deren relativer Lage zueinander, insbesondere durch Drehung des Aufnahmeabschnitts 16 relativ zu dem Befestigungselement 22, die Klemmkraft und/oder der Abstand von Aussparungsgrund zu Befestigungselement 22 kontinuierlich eingestellt werden kann.

Im dargestellten Fall umfasst die Verankerungsvorrichtung ein in Form einer einteiligen Knochenschraube ausgebildetes Verankerungselement 10. Die Knochenschraube weist einerseits den in Form eines Schraubenschafts ausgebildeten Verankerungsabschnitt 12 auf, wobei der Schraubenschaft eingerichtet ist, skelettal mittels dessen Außengewindes 20 befestigt zu werden. Anderseits weist die Knochenschraube den in Form eines an den Schraubenschaft angrenzenden Schraubenkopfs ausgebildeten Befestigungsabschnitt 14 auf, der eingerichtet ist, mit dem zumindest einen kieferorthopädischen Element gekoppelt zu werden. Dabei ist der Schraubenkopf mit der Befestigungseinrichtung vorgesehen ist, die derart ausgebildet ist, dass das kieferorthopädische Element zumindest abschnittsweise zwischen einer als Aufnahmeabschnitt fungierenden Aussparung (bzw. Schlitz) des Schraubenkopfs und einer auf ein Außengewinde 18 des Schraubenkopfs aufgeschraubten und als Befestigungselement fungierenden Mutter mit kontinuierlich einstellbarer Klemmkraft verklemmt werden kann. Wie den Figuren 1 bis 4 insbesondere entnommen werden kann, ist die Aussparung in dem Schraubenkopf als durch den Schraubenkopf durchgängiger Schlitz ausgebildet ist, wobei eine Aussparungstiefenrichtung parallel zur Schraiabenachse verläuft und die Aussparung geradlinig durch die Schraubenachse, d.h. mittig durch den Schraubenkopf, verläuft. Insbesondere ist der Schraubenkopf dergestalt, dass er weiterhin einen Absatz gegenüber dem Schraubenschaft ausbildet und teilweise zylindrisch ausgestaltet ist, wobei die Zylinderachse mit der Schraubenachse zusammenfällt. Wie vorstehend bereits erwähnt ist, ist der zylindrische Schraubenkopf mit dem Außengewinde 18 vorgesehen, um die Schraubenmutter mit entsprechendem Innengewinde auf den Schraubenkopf aufdrehen bzw. aufschrauben zu können. Der Absatz ist so ausgebildet, dass der Absatz form- und/oder kraftschlüssig mit einem Werkzeug zum Drehen der Knochenschraube in Kontakt gebracht werden kann. Im dargestellten Fall ist der Absatz als Achtkantabsatz ausgebildet.

Bestimmungsgemäß wird die Knochenschraube zunächst skelettal mittels deren Verankerungsabschnitt 12, insbesondere dessen Außengewindes, befestigt, beispielsweise an einem Ober- oder Unterkiefer, nämlich durch Ansetzen eines Werkzeugs an dem Absatz, beispielweise eines kieferorthopädischen Montagewerkzeugs wie ein Schraubschlüssel, insbesondere ein Ring-, Gabel- oder Maulschlüssel, ein Steck- oder Rohrschlüssel etc., und Drehen des Werkzeugs. Danach wird das kieferorthopädische Element, beispielsweise ein Zahnspangenrunddraht in die Aufnahme bzw. Aussparung des Schraubenkopfes der Knochenschraube eingesetzt und mittels aufdrehen der Schraubenmutter mit einer engtsprechenden Klemmkraft verklemmt.

Bevorzugt weist das als Knochenschraube ausgebildete Verankerungselement 10 eine axiale Gesamtlänge auf, die zwischen 11 mm und 17 mm liegt, wobei dabei vorzugsweise der gesamte Schraubenkopf (samt Befestigungsabschnitt und Absatz) eine Länge von 3 mm und der als Schraubenschaft ausgebildete Verankerungsabschnitt eine Gesamtlänge, die zwischen 8 mm und 14 mm liegt, in Axialrichtung aufweisen. Vorzugsweise weist der Absatz dabei eine Länge von 0,8 mm in Axialrichtung auf. Der Schlitz ist vorzugsweise 1,2 mm breit und 2,6 mm in Axialrichtung tief, wobei der Absatz vorzugsweise einen Durchmesser von 3,2 mm von einer durch zwei Kanten begrenzten Absatzumfangsfläche zu einer gegenüberliegenden Absatzumfangsfläche hat.

Besonders bevorzugte Paarungen aus Knochenschraubengesamtlänge/Schraubenschaftgesamtlänge sind folgende: 11 mm/8 mm; 13 mm/10 mm; 15 mm/12 mm; 17 mm/14 mm.

Die in der vorstehenden Beschreibung, in den Zeichnungen sowie in den Ansprüchen offenbarten Merkmale der Erfindung können sowohl einzelnen als auch in beliebiger Kombination für die Verwirklichung der Erfindung wesentlich sein.

## Patentansprüche

1. Skelettale Verankerungsvorrichtung zur Befestigung zumindest eines kieferorthopädischen Elements zur Verwendung für eine kieferorthopädische Behandlung, wobei die Verankerungsvorrichtung ein Verankerungselement (10) umfasst, das einen Verankerungsabschnitt (12) aufweist, der eingerichtet ist, skelettal an einem Ober- oder Unterkieferknochen des Menschen befestigt zu werden, und weiterhin einen Befestigungsabschnitt (14) aufweist, der eingerichtet ist, mit dem zumindest einen kieferorthopädischen Element gekoppelt zu werden, wobei der Befestigungsabschnitt (14) mit einer Befestigungseinrichtung vorgesehen ist, die derart ausgebildet ist, dass das kieferorthopädische Element zumindest abschnittsweise mit kontinuierlich einstellbarer Klemmkraft an dem Befestigungsabschnitt (14) verklemmt werden kann.

2. Skelettale Verankerungsvorrichtung gemäß Anspruch 1, wobei die Befestigungseinrichtung derart ausgebildet ist, dass das kieferorthopädische Element zwischen einem Aufnahmeabschnitt (16) des Befestigungsabschnitts (14) und einem mit dem Befestigungsabschnitt (14) koppelbaren Befestigungselement (22) verklemmt werden kann, wobei der Aufnahmeabschnitt (16) und das Befestigungselement (22) so ausgebildet sind, dass in Abhängigkeit von einer Änderung von deren relativer Lage zueinander die Klemmkraft kontinuierlich eingestellt werden kann.

3. Skelettale Verankerungsvorrichtung gemäß Anspruch 2, wobei der Aufnahmeabschnitt (16) und das mit dem Befestigungsabschnitt (14) koppelbare Befestigungselement (22) so ausgebildet sind, dass die Klemmkraft durch Drehung des Aufnahmeabschnitts (16) relativ zu dem Befestigungselement (22) kontinuierlich eingestellt werden kann.

4. Skelettale Verankerungsvorrichtung gemäß einem der Ansprüche 1 bis 3, wobei die Verankerungsvorrichtung ein in Form einer Knochenschraube ausgebildetes Verankerungselement (10) umfasst, das einen in Form eines Schraubenschafts ausgebildeten Verankerungsabschnitt (12) aufweist, der eingerichtet ist, skelettal, vorzugsweise mittels eines Außengewindes (20), befestigt zu werden, und weiterhin einen in Form eines an den Schraubenschaft angrenzenden Schraubenkopfs ausgebildeten Befestigungsabschnitt (14) aufweist, der eingerichtet ist, mit dem zumindest einen kieferorthopädischen Element gekoppelt zu werden, wobei der in Form des Schraubenkopfes ausgebildete Befestigungsabschnitt (14) mit einer Befestigungseinrichtung vorgesehen ist, die derart ausgebildet ist, dass das kieferorthopädische Element zumindest abschnittsweise zwischen einer als Aufnahmeabschnitt fungierenden Aussparung des Schraubenkopfs und einer auf ein Außengewinde (18) des Schraubenkopfs aufgeschraubten und als Befestigungselement fungierenden Mutter mit kontinuierlich einstellbarer Klemmkraft verklemmt werden kann.

5. Skelettale Verankerungsvorrichtung gemäß einem der AnSprüche 1 bis 4, wobei die Aussparung in dem Schraubenkopf als durch den Schraubenkopf durchgängiger Schlitz ausgebildet ist und/oder die Aussparungstiefenrichtung parallel zur Schraubenachse verläuft und/oder die Aussparung geradlinig durch die Schraubenachse verläuft und/oder die Aussparung mittig durch den Schraubenkopf verläuft.
